# EUROPEAN PATENT SPECIFICATION

(11) **EP 0 949 938 B1**
(45) Date of publication and mention of the grant of the patent: **17.12.2003**
(21) Application number: 97943687.0
(22) Date of filing: 07.10.1997
(51) Int. Cl.: A61N 2/02

(54) **MAGNETOTHERAPY DEVICE**
MAGNETOTHERAPEUTISCHE VORRICHTUNG
DISPOSITIF DE MAGNETOTHERAPIE

(30) Priority: 12.07.1997 BY 971248
(43) Date of publication of application: 20.10.1999
(73) Proprietor: Pletnev, Sergey Vladimirovich, Minsk, 220070 (BY)
(72) Inventor: Pletnev, Sergey Vladimirovich, Minsk, 220070 (BY)
(74) Representative: Kovacs, Paul
(86) International application number: BY9700006
(87) International publication number: WO99002217

(56) References cited:
- WO-A-91/02501
- WO-A-94/27675
- WO-A-96/11723
- DE-U- 8 814 357
- GB-A- 2 270 000
- GB-A- 2 295 093

## Description

The present invention relates to the medical field, and in particular to magnetotherapy of various diseases and disorders of human and other living beings. The device according to the invention may be applied for therapy and prophylaxis of various diseases and disturbances.

The prior art discloses methods for therapeutic treatment of a human organism by means of a magnetic field of HF electromagnetic oscillations producing a thermal effect /1/. Such methods are commonly used in practice at physiotherapeutic rooms. However as they generally use only the heating effect so in some cases such methods may be counterindicated.

The prior art discloses methods of bioacoustic stimulation and music therapy consisting in producing an acoustic effect on the human organism /2/. In the said methods a human organism is subjected to by a harmonious sound effect with the purpose of producing a synchronizing effect. These approaches demonstrate immediate effecting the internal organs whereas timbres of various music instruments have 'certain congeniality to corresponding physiological systems" /p. 23/.

The prior art discloses methods of therapy using acoustic vibrations, such as vocal therapy /3/. Acoustic vibrations of certain organs were measured in the course of vocal therapy. It was found out that each organ has its specific "resonance' note which may actuate in it maximum vibration fluctuations.

Nevertheless, the said methods are of low effectiveness, since they have no immediate effect on biochemical and metabolic processes in cells and may be generally used for prophylaxis.

The prior art discloses methods for magnetotherapy consisting in treating various human organs by means of pulsed or variable LF magnetic filed while the choice of the specific magnet in the form of a catheter, solenoid, etc. is determined by the cured organ /2/. These methods provide local treatment of a specific organ but do not specify characteristics of such treatment.

The prior art discloses a magnetotherapy method /1/ for treatment of gynecological diseases wherein a biological object is irradiated by pulse magnetic LF field movable in time and space (on analogy to the travelling wave) being in coincidence with contraction of the cardiac muscle.

The apparatus for embodiment of the said method generates packets of pulse magnetic fields at the field generating rate per packet optimally coinciding with the cardiac contraction rate, and being synchronized with the latter, while the value of magnetic induction at each treated point of the biological object varies from 0 up to maximum 40-60 mT sliding down to 0 again, representing thereby one of the most important therapeutic factors.

According to the said embodiment a packet of pulse magnetic fields is discretely relocated for a distance corresponding to the interval between field axes within a packet.

The prior art also discloses method /2/, similar to the said one, used for treatment of patients suffering from ishemic heart desease which consists in synchronizing the travelling magnetic field to the cardiac cycle.

Therapy under such methods generally is carried out in series for certain periods of time which duration is usually specified by a physician. However herewith individual characteristics of an organ and an organism as a whole are not considered during the treatment procedure and thereby the effectiveness of therapeutic treatment is lowered and moreover maximum favorable medical effect is not achieved.

A device simultaneously applying an acoustical signal and a magnetic field modulated by the acoustical signal is known from document C1 106 80 47.

According to the invention there is provided a device for magnetotherapy as defined in claim 1.

It was found out that simultaneous administration of two therapeutic means- pulse magnetic and acoustic means- along with activation of biochemical processes in the tissue of the treated organ and administration of the synchronizing acoustic effect, rises sharply the effectiveness of therapeutic treatment and allows to cure some diseases incurable particularly by each one of these means. Moreover the acoustic effect may be imposed as on the total organism through listening so as immediately on the treated organ. Besides, this allows to reduce substantial the effective parameter of magnetic filed diminishing thereby probability of side effects due to overdosing and reducing energy consumption during therapeutic treatment. Moreover, in case the acoustic effect is imposed on the total organism, i.e. through the organ of hearing, then the favorable effect on the central nervous system produces additional positive result and reduces the time for rehabilitation of the organ function.

Further advantages of the invention are that for the acoustic effect the harmonious sounding is applied, in particular music speech producing the stimulating effect on the function of the treated organ.

In accordance with further objects of the invention the impulse amplitude of magnetic field is modulated by an acoustic signal.

According to the invention the electromagnetic response of the organ is measured and then the harmonic component of the organ electromagnetic response frequency is selected as the parameter determining impulse frequency of the magnetic field.

In accordance with the invention the frequency of pulse packets repetition matches the music speech rhythm and the frequency of pulse repetition in a packet matches the harmonic sound spectrum.

Therapeutic treatment is administrated at least in two steps, while at the first (preparatory) step the music treatment producing a sedative and relaxing effect is imposed. At the second (therapeutic) step there is applied a music effect stimulating the function of the treated organ.

At the final (prophylactic) step there is applied a music effect stimulating the function of the treated organ, whereupon magnetic field effect is gradually reduced up to the full turn-off.

In accordance with further objects of the invention concurrently with the magnetic and acoustic effect a treated organ is subjected to the vibrating effect.

The frequency of the vibrating effect or/and the frequency of pulse packets repetition matches the low frequency harmonic components of the acoustic effect and the frequency of pulse repetition in a packet matches the high frequency harmonic components of the acoustic effect.

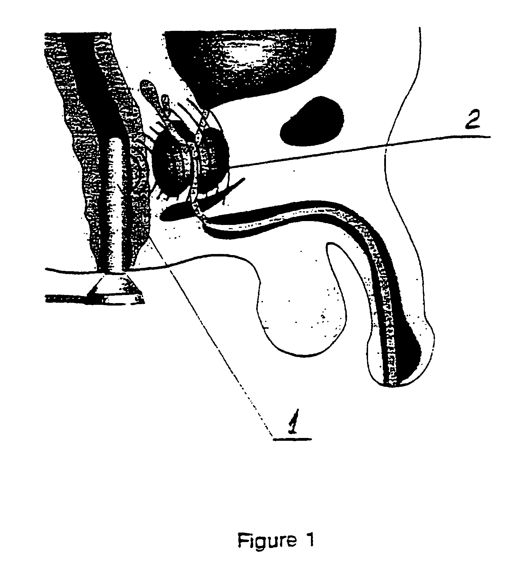

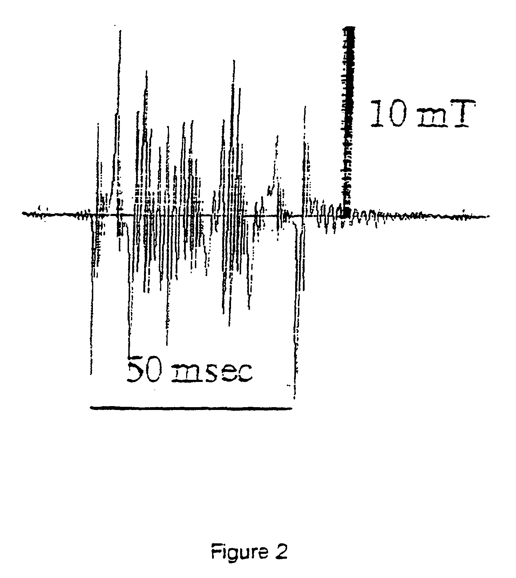

The example of an embodiment of the device of the present invention is illustrated by Figures 1 and 2.
Figure 1 is a diagram of therapeutic treatment of prostate.
Figure 2 shows shape and parameters of the magnetic signal effect under the invention.

### Example.

In accordance with one embodiment of the invention during treatment of virilia diseases and, in particular impotence, the prostate is effected by the pulse magnetic field. The therapeutic magnetic inductor 1 (introduced rectally) is positioned in the rectum so that therapeutic magnetic field is directed to the prostate 2. In the course of therapeutic treatment the organism is subjected to an acoustic effect of a harmonious music sounding by way of listening to a corresponding music melody. At the same time pulse magnetic field is modulated by an acoustic signal while the frequency of pulse packets repetition matches one of the musical rhythms and the frequency of impulse repetition in a packet matches one of the harmonic sound components with allowance for the stimulating effect of the component on a corresponding organ. In order to achieve a more fine adjustment with the organ, an electromagetic response of an organ is measured and the magnetic signal is modulated according to the measured response. For this purpose a corresponding detector is placed on the inductor or near the organ. According to one embodiment of the invention the detector measuring the electrical impedance is introduced as a probe through urethra and the signal is registered by the broadband amplifier. The signal format and the amplitude of magnetic effect are shown at Figure 2. It is obvious that the signal format on the Figure 2 looks like the signal format of the musical note "do".

In the course of investigations the therapeutic procedure was administrated in three steps. At the first preparatory step magnetotherapy was administrated under general sedative and relaxing effect. At the second step a stimulating and galvanic music was used along with the corresponding modulation of the magnetic field.

At the third step the amplitude of the magnetic field was gradually reduced to zero under continued musical effect. On exposure to such effects normalization and mending of the prostate function was observed. In this case the normalizing effect manifested itself afterwards even out of clinic in the absence of the magnetic effect so that a normal erection took place under the music effect, etc.

A vibrating effect may be produced in addition to the magnetic and acoustic effect. The spectrum of this effect may occupy the low frequency component of the music effect or the rhythm only. This effect also contributes to the therapeutic action by increasing it.

Thus complex and effective magnetic therapeutic treatment provided by the device of the invention without involving any surgical or biochemical intervention allows to obtain results which can not be achieved by separate means while accompanying pharmaceutical preparations may be administrated on doctor's order.

### Sources of information

1. Physiotherapy Manual ( edited by V. G. Yasnogorodski) Moscow, Medicine, 1992, p. 13.
2. 2^{nd} International Conference " Theoretical and clinical aspects of bioresonace and multiresonace therapy. Abstracts and reports. IMRDIZ editor, Moscow, 1996, p.21-25.
3. 2^{nd} International Conference " Theoretical and clinical aspects of bioresonace and multiresonace therapy. Abstracts and reports. IMRDIZ editor, Moscow, 1996, p.25-30.
4. Method of pulse magnetotherapy of biological objects and device therefore. RU94009616, A61 N 2/00
5. Method of treatment of patients suffering from chronic ischemic heart decease SU 1537276, A61N2/00.

## Claims

1. A device for magnetotherapy, comprising a magnetic field inductor and means for producing an acoustic signal in the form of a musical sound, **characterized in that** the magnetic field inductor is adapted for being positioned in the vicinity of said organ and is connected to a generator of packets of electric pulses for creating a pulsed magnetic field consisting of packets of pulses, and **in that** the device further comprises means for modulating, by said acoustic signal, the amplitude of pulses of the magnetic field generated by said inductor, wherein the magnetic field inductor is adapted to generate pulses of magnetic field having a frequency of repetition of packets of pulses matching a musical sound rhythm, while the frequency of pulses, in each such packet of pulses, matches the musical sound harmonic spectrum; and
**in that** the device further comprises means for detecting an electromagnetic response of the organ subjected to the therapeutic treatment, said response being measured upon subjecting the organ to the magnetic field generated by said inductor, and means for selecting a harmonic frequency component of the electromagnetic response as a parameter determining the frequency of pulses of the magnetic field.

2. The device according to claim 1, **characterized in that** it is arranged such that, during therapeutic treatment, the inductor can be applied in close vicinity of the organ to be treated and the means for producing the acoustic signal can be applied to the patent's body, said acoustic signal being generated according to at least two modes, including a first preparatory mode during which musical sounds, producing a sedative and/or relaxing effect, are applied.

3. The device according to claim 2, **characterized in that** the means for producing the acoustic signal is arranged such that it generates, during a second, therapeutic, mode, musical sounds which produces a simulating effect on the functions of the treated organ.

4. The device according to any of claims 1 to 3, **characterized in that** it is arranged such that the therapeutic treatment can be administrated according to at least three modes, including a final, prophylactic, mode during which musical sounds, which produce a simulating effect on the functions of the treated organ, are applied, followed by a gradual reduction of the magnetic field to full turn-off.

5. The device according to any of claims 1 to 4, **characterized in that** it further comprises vibrating means adapted for being applied to the treated organ.

6. The device according to claim 5, **characterized in that** it is arranged such that the pulse frequency of the vibrating means, and/or the frequency of repetition of packets of pulses of magnetic field, is adjustable to the low frequency harmonic component of the acoustic signal and the frequency of pulses of magnetic filed, in each pulse packet, is adjustable to the high frequency harmonic component of the acoustic signal.

## Patentansprüche

1. Vorrichtung zur Magnetotherapie, umfassend einen Magnetfeld-Induktor und Mittel zum Erzeugen eines akustischen Signals in der Art eines Musiktons, **dadurch gekennzeichnet, dass** der Magnetfeldinduktor angepasst ist um in der Nähe eines Organs positioniert zu sein und mit einem Generator für Pakete von elektrischen Pulsen verbunden ist zum Erzeugen eines gepulsten magnetischen Feldes, das Pakete von Pulsen umfasst, und dass die Vorrichtung ferner Mittel zum Modulieren der Amplitude der Pulse des durch den Induktor erzeugten magnetischen Feldes mittels des akustischen Signals umfasst, wobei der Magnetfeldinduktor angepasst ist um Pulse eines magnetischen Feldes zu erzeugen mit einer Wiederholrate der Pakete der Pulse, welche an einen Musiktonrhythmus angepasst ist, während die Frequenz der Pulse in jedem eines solchen Paketes von Pulsen an die Oberwellen des Musiktons angepasst ist; und
dass die Vorrichtung ferner Mittel zum Ermitteln einer elektromagnetischen Antwort des Organs, welches dem therapeutischen Verfahren unterworfen ist, umfasst, wobei die Antwort gemessen wird auf das Unterwerfen des Organs dem durch den Induktor erzeugten magnetischen Feldes, und Mittel zum Auswählen einer Oberwellenkomponente der elektromagnetischen Antwort als ein Parameter zum Festsetzen der Frequenz der Pulse des magnetischen Feldes.

2. Vorrichtung nach Anspruch 1, **dadurch gekennzeichnet, dass** diese während der therapeutischen Behandlung derart eingerichtet ist, dass der Induktor in direkter Nachbarschaft zu dem zu behandelnden Organ angewendet werden kann und das Mittel zum Erzeugen des akustischen Signals an dem Patientenkörper angewendet werden kann, wobei das akustische Signal wenigstens in zwei Formen erzeugt wird, umfassend eine erste vorbereitende Form während der Musiktöne angewendet werden, die eine beruhigende und/oder entspannende Wirkung erzeugen.

3. Vorrichtung nach Anspruch 2, **dadurch gekennzeichnet, dass** das Mittel zum Erzeugen des akustischen Signals derartig eingerichtet ist, dass es während einer zweiten therapeutischen Form musikalische Töne erzeugt, die eine stimulierende Wirkung auf die Funktion des behandelnden Organs ausübt.

4. Vorrichtung nach einem der Ansprüche 1 bis 3, **dadurch gekennzeichnet, dass** es derartig eingerichtet ist, dass die therapeutische Behandlung gemäß wenigstens drei Formen ausgeübt werden kann, umfassend eine letzte prophylaktische Form während der musikalische Töne angewendet werden, die einen stimulierenden Effekt auf die Funktion des behandelten Organs erzeugt, gefolgt von einer schrittweisen Verminderung des magnetischen Feldes bis zum vollständigen Ausschalten.

5. Vorrichtung nach einem der Ansprüche 1 bis 4, **dadurch gekennzeichnet, dass** es ferner ein Schwingungsmittel umfasst, das zum Anwenden an dem zum behandelnden Organ angepasst ist.

6. Vorrichtung nach Anspruch 5, **dadurch gekennzeichnet, dass** es derartig eingerichtet ist, dass die Pulsfrequenz des Schwingungsmittels und/oder die Wiederholrate der Pakete der Pulse des magnetischen Feldes einstellbar ist auf eine niedrige Oberwellenkomponente des akustischen Signals und die Frequenz der Pulse des magnetischen Feldes in jedem Pulspaket einstellbar ist auf eine hohe Oberwellenkomponente des akustischen Signals.

## Revendications

1. Dispositif de magnétothérapie, comprenant un inducteur de champ magnétique et des moyens pour produire un signal acoustique sous forme d'un son musical, **caractérisé en ce que** l'inducteur de champ magnétique est agencé de manière à pouvoir être disposé au voisinage d'un organe et connecté à un générateur de trains d'impulsions électriques pour engendrer un champ magnétique modulé en impulsions, constitué de trains d'impulsions, et **en ce que** le dispositif comprend en outre des moyens pour moduler, par ledit signal acoustique, l'amplitude des impulsions du champ magnétique engendré par ledit inducteur, l'inducteur de champ magnétique étant agencé de manière à permettre d'engendrer des impulsions de champ magnétique ayant une fréquence de répétition de trains d'impulsions assortie à un rythme de son musical, la fréquence des impulsions, dans chaque train d'impulsions, étant assortie au spectre d'harmoniques du son musical; et **en ce que** le dispositif comprend, en outre des moyens pour détecter une réponse électromagnétique de l'organe soumis au traitement thérapeutique, ladite réponse étant mesurée tout en exposant l'organe au champ magnétique engendré par ledit inducteur, et des moyens pour effectuer la sélection d'un élément constitutif d'une fréquence harmonique de la réponse électromagnétique en tant que paramètre déterminant la fréquence des impulsions du champ magnétique.

2. Dispositif selon la revendication 1, **caractérisé en ce qu'**il est agencé de manière à permettre d'appliquer, au cours du traitement thérapeutique, l'inducteur au voisinage immédiat de l'organe à traiter, et les moyens pour produire le signal acoustique au corps du patient, ledit signal acoustique étant engendré selon au moins deux modes, comprenant un premier mode préparatoire au cours duquel des sons musicaux, produisant un effet sédatif ou reposant, sont appliqués.

3. Dispositif selon le revendication 2, **caractérisé en ce que** les moyens pour produire le signal acoustique sont agencés de façon à engendrer, au cours d'un second mode, thérapeutique, des sons musicaux produisant un effet stimulant les fonctions de l'organe traité.

4. Dispositif selon l'une quelconque des revendications 1 à 3, **caractérisé en ce qu'**il est agencé de manière à permettre d'administrer le traitement thérapeutique selon au moins trois modes, comprenant un mode final, prophylactique, au cours duquel des sons musicaux, produisant un effet stimulant les fonctions de l'organe traité, sont appliqués, suivi par une réduction progressive du champ magnétique jusqu'à l'interruption complète de celui-ci.

5. Dispositif selon l'une quelconque des revendications 1 à 4, **caractérisé en ce qu'**il comprend, en outre, des moyens vibrateurs agencés de manière à pouvoir être appliqués à l'organe à traiter.

6. Dispositif selon la revendication 5, **caractérisé en ce qu'**il est agencé de manière que la fréquence d'impulsions des moyens vibrateurs et/ou la fréquence de répétition des trains d'impulsions soient réglables en accord avec l'élément constitutif de basse fréquence harmonique du signal acoustique et que la fréquence d'impulsions du champ magnétique, dans chaque train d'impulsion, soit réglable en accord avec l'élément constitutif de haute fréquence harmonique du signal acoustique.
